# EUROPÄISCHE PATENTSCHRIFT

(11) **EP 1 758 649 B1**
(45) Veröffentlichungstag und Bekanntmachung des Hinweises auf die Patenterteilung: **27.05.2009**
(21) Anmeldenummer: 05791916.9
(22) Anmeldetag: 19.09.2005
(51) Int. Cl.: A61N 5/10

(54) **MEDIZINISCHE STRAHLENTHERAPIEANORDNUNG**
MEDICAL RADIOTHERAPY ASSEMBLY
ENSEMBLE MEDICAL DE RADIOTHERAPIE

(30) Priorität: 30.09.2004 DE 102004048216; 23.12.2004 DE 102004062473
(43) Veröffentlichungstag der Anmeldung: 07.03.2007
(73) Patentinhaber: SIEMENS AKTIENGESELLSCHAFT, 80333 München (DE)
(72) Erfinder: HERRMANN, Klaus, 90403 Nürnberg (DE)
(86) Internationale Anmeldenummer: PCT/EP2005/054665
(87) Internationale Veröffentlichungsnummer: WO 2006/034973

(56) Entgegenhaltungen:
- EP-A- 0 220 501
- EP-A- 1 358 908
- DE-A1- 19 625 407
- DE-A1- 19 958 864
- DE-A1- 19 963 440
- DE-U- 20 109 313
- US-A1- 2004 024 300
- PATENT ABSTRACTS OF JAPAN Bd. 1997, Nr. 02, 28. Februar 1997 (1997-02-28) & JP 08 266650 A (MITSUBISHI ELECTRIC CORP), 15. Oktober 1996 (1996-10-15)
- KAMADA T ET AL: "A horizontal CT system dedicated to heavy-ion beam treatment" RADIOTHERAPY AND ONCOLOGY ELSEVIER IRELAND, Bd. 50, Nr. 2, Februar 1999 (1999-02), Seiten 235-237, XP002359765 ISSN: 0167-8140
- KATUIN ET AL: 'The Use of Industrial Robot Arms for High Precision Patient Positioning' CAARI 2002 CONFERENCE 2002, DENTON, XP002456988

## Beschreibung

Die Erfindung betrifft eine medizinische Strahlentherapieanordnung, wie sie zur Behandlung von Tumoren eingesetzt wird.

Bei der Strahlentherapie mit Schwerionen werden mit Hilfe von beschleunigten Partikeln Tumore beschossen. Ein Teilchenbeschleuniger erzeugt hierbei einen Partikelstrahl mit einem im Raum feststehenden Strahlaustritt. Zur Behandlung muss der Patient in eine exakte, vordefinierte Position gebracht werden und zwar derart, dass sich der Tumor im so genannten Isozentrum des Partikelstrahls befindet. In Abhängigkeit der Position des Tumors im Körper werden unterschiedliche Bestrahlungspositionen (Felder) für den Patienten vorgesehen, beispielsweise wird der Patient in liegender oder auch in sitzender Position vor dem Partikelstrahler positioniert.

Im Vorfeld der Strahlentherapie ist es daher erforderlich, die Position des Tumors möglichst exakt zu bestimmen. Hierzu werden bildgebende Diagnoseverfahren eingesetzt. Röntgen-Diagnosegeräte sind beispielsweise zu entnehmen aus der DE 189 55 213 C2, DE 189 27 022 C2 oder aus EP 0 220 501 B1. Die Tumor-Position wird üblicherweise durch äußere Markierungen direkt am Patienten, so genannte Hautmarkierungen, oder auch an sogenannten Immobilisierungsmasken angezeigt.

Abhängig von der Tumorart und Größe wird in der Therapieplanung die Anzahl der einzelnen Bestrahlungen (Fraktionen) festgelegt, die zur sicheren Zerstörung des Tumors notwendig sind. Üblicherweise werden pro Patient 20-30 Bestrahlungen über einen Zeitraum von mehreren Wochen durchgeführt.

Zum Bestrahlen wird der Patient in einen Bestrahlungsraum mit einem Strahlenaustrittsfenster für den Partikelstrahl gebracht. Der Patient wird auf einer Patientenliege immobilisiert oder fixiert. Unter Patientenliege wird hier allgemein sowohl eine Liege im engeren Sinn als auch ein Stuhl verstanden, auf denen der Patient immobilisiert ist. Anschließend wird der Patient entsprechend den an der Haut oder Immobilisierungsmasken befindlichen Markierungen in das bekannte und über Laser markierte feststehende Isozentrum verfahren, bevor mit der Strahlentherapie begonnen wird. Mitunter ist im Bestrahlungsraum zusätzlich zum Strahlenaustrittsfenster ein feststehendes bildgebendes Gerät vorgesehen, mit dem die Tumorposition anhand anatomischer Landmarken verifizierbar ist. Nach der Verifikation wird der Patient von seiner Aufnahmeposition in eine Bestrahlungsposition verfahren.

Lageveränderungen des Tumors zwischen dem Zeitpunkt der Diagnose und dem Zeitpunkt der jeweiligen Bestrahlung können zu einer Beeinträchtigung der Wirksamkeit der Strahlentherapie führen. Auch ist die Ausrichtung des Patienten in das Isozentrum anhand der Markierungen mit Ungenauigkeiten behaftet. Selbst wenn unmittelbar vor der Bestrahlung die Tumorposition verifiziert wird, ist aufgrund des Verfahrens des Patienten von der Aufnahmeposition in die Bestrahlungsposition eine exakte Positionierung schwierig.

Die Schrift Katuin JE et al, "The Use of Industrial Robot Arms for High Precision Patient Positioning", CAARI Conference, Denton, 2002, zeigt eine Patientenliege, die von einem Roboterarm gehalten wird. An einem weiteren Roboterarm ist ein Panel befestigt, mit dem Röntgenstrahlen, die von einer statischen Röntgenquelle ausgehen, detektiert werden.

Der Erfindung liegt die Aufgabe zugrunde, eine medizinische Strahlentherapieanordnung anzugeben, mit der eine möglichst effiziente Strahlentherapie ermöglicht ist.

Die Aufgabe wird gemäß der Erfindung gelöst durch eine Strahlentherapieanordnung, insbesondere Ionenstrahltherapieanordnung, gemäß Patentanspruch 1. Diese umfasst ein Austrittsfenster für einen Partikelstrahl mit einem im Raum feststehenden Strahlaustritt sowie eine Patientenlagerungs-vorrich - tung mit einer Patientenliege, die vor das Austrittsfenster in eine zur Bestrahlung eines Patienten geeignete Bestrahlungsposition bringbar ist. Die Anordnung weist zudem ein Diagnose- oder ein bildgebendes Gerät zur Bestimmung oder Verifikation der Position eines zu bestrahlenden Tumors auf. Das bildgebende Gerät ist insbesondere ein Röntgengerät und umfasst eine Röntgenstrahlenquelle sowie einen dieser genüberliegenden Röntgenstrahlendetektor. Das bildgebende Gerät ist im Raum um die in der Bestrahlungsposition befindliche Patientenliege verfahrbar. Hierzu sind die Röntgenstrahlenquelle und der Röntgenstrahlendetektor an einem gemeinsamen, insbesondere mechanisch steifen Tragarm befestigt. Durch diese Maßnahme ist daher gewährleistet, dass Röntgenstrahlenquelle und Röntgenstrahlendetektor jeweils in der gleichen Stelle zueinander positioniert sind. Weiterhin braucht zur Bewegung im Raum lediglich der Tragarm entsprechend angesteuert und verfahren zu werden. Es sind also keine zwei unabhängigen Bewegungen im Raum erforderlich, wodurch die Ansteuerung einfach gehalten ist. Um bei jeder möglichen Bestrahlungsposition des Patienten eine bildgebende Diagnose zu ermöglichen, ist der Tragarm derart gelagert und ansteuerbar, dass sowohl eine Angularbewegung um die durch den Partikelstrahl definierte Längsachse als auch eine Orbitalbewegung um eine zur Längsachse senkrechte Achse ausführbar ist. Durch Überlagerung dieser beiden Dreh-Freiheitsgrade in Kombination mit einer zur einen Seite offenen Ausbildung des Tragarms lassen sich sämtliche Diagnosepositionen zur Verifikation des Tumors einnehmen, unabhängig von der jeweiligen Position des Patienten.

Ein besonderer Vorteil dieser Strahlentherapieanordnung ist darin zu sehen, dass der Partikelstrahler mit einem bildgebenden Gerät derart kombiniert ist, dass der auf der Patientenliege befindliche Patient in ein und derselben Position sowohl einer Bildaufnahme als auch der Therapie unterzogen werden kann. D.h. es ist unmittelbar in der Bestrahlungsposition eine Bestimmung oder Verifikation der Position des Tumors ermöglicht. Die Bildaufnahme wird unmittelbar vor oder auch während der Bestrahlung durchgeführt. Es ist keinerlei Umlagerung des Patienten von der Bildaufnahme zur Bestrahlung erforderlich, welche immer die Gefahr einer Verschiebung des Tumors in sich birgt. Durch die Verifikation der Position des Tumors in der Bestrahlungsposition ist daher eine exakte Positionierung des Tumors in das Isozentrum des Partikelstrahlers ermöglicht, so dass insgesamt die Strahlentherapie hocheffizient eingesetzt werden kann. Aufgrund der freien Verfahrbarkeit des bildgebenden Geräts im Raum ist zudem unerheblich, in welcher Position des Patienten, ob in sitzender oder in liegender Position, die Strahlentherapie erfolgt.

Ein mit dieser Strahlentherapieanordnung durchzuführendes Verfahren zeichnet sich demnach dadurch aus, dass in der jeweiligen Therapieposition eine Positionsverifikation zur Bestimmung der Tumorposition erfolgt. Die Positionsverifikation ist durch die spezielle Ausgestaltung der Strahlentherapieanordnung hierbei in jeder beliebigen Therapieposition des Patienten ermöglicht. Zur Positionsverifikation wird vorzugsweise zum einen die Tumorposition anhand von 2-dimensionalen Projektionsaufnahmen bestimmt. In einer vorteilhaften Alternative wird die Positionsverifikation zur Bestimmung der Tumorposition anhand von 3-dimensionalen Schnittbildaufnahmen durchgeführt.

Von wesentlicher Bedeutung ist weiterhin, dass die Positionsverifikation als auch die eigentliche Bestrahlung sowohl in liegender als auch in sitzender Stellung des Patienten und damit in jeder beliebigen Therapieposition des Patienten ermöglicht ist. Gemäß einer zweckdienlichen Weiterbildung ist daher vorgesehen, dass die Patientenliege als Liege für eine liegende Bestrahlungsposition oder als Stuhl für eine sitzende Bestrahlungsposition des Patienten ausgebildet oder umbaubar ist. Durch die hier beschriebene Anordnung ist es daher möglich, bei einem ortsfesten Strahlaustritt den Patienten in beliebigen Bestrahlungspositionen zu Bestrahlen. Insbesondere ist aufgrund der Möglichkeit der sitzenden Position auch eine frontale Bestrahlung ermöglicht. Diese durch die spezielle Anordnung bedingte Variabilität ermöglicht im Vergleich zu bisherigen Systemen erhebliche Kosteneinsparungen, da insbesondere auf eine sogenannte Gantry verzichtet werden kann, mit deren Hilfe der Bestrahlungswinkel des Teilchenstrahls in aufwändiger und damit kostenintensiver Weise eingestellt wird.

Ein weiterer besonderer Vorteil der gerätetechnischen Kombination des Partikelstrahlers mit dem bildgebenden Gerät ist darin zu sehen, dass im Verlauf einer Bestrahlung die Position des Tumors verifiziert oder kontrolliert und damit eine aktive Lagekontrolle des Patienten durchgeführt werden kann und auch wird. Auch hierzu werden mit Hilfe des bildgebenden Geräts beispielsweise 2D-Projektionsaufnahmen durchgeführt und die ermittelten Bilder werden mit Hilfe einer geeigneten Bildauswertung mit den zuvor ermittelten 3D-Bildern des Tumors verglichen. Dadurch ist es möglich, online, also während einer Bestrahlung, auf Positionsveränderungen des Tumors durch eine insbesondere automatisch gesteuerte Verschiebung des Patienten in die optimale Bestrahlungsposition zu reagieren.

Insgesamt ist durch die sehr flexible Positionierbarkeit des Diagnosegeräts ermöglicht, mit ein und demselben bildgebenden Gerät, d.h. mit ein und demselben Strahlenquelle-Strahlendetektor-Paar, für die verschiedensten Bestrahlungspositionen Bildaufnahmen des Tumors zu erzeugen. Im Vergleich zu feststehenden Röntgen-Diagnosegeräten, die jeweils nur für eine Patientenposition zur Bilderzeugung vorgesehen sind, ist dadurch auch eine deutliche gerätetechnische Vereinfachung und kostengünstigere Lösung erzielt.

Als Röntgengerät wird hierbei vorzugsweise ein konventionelles Röntgengerät beispielsweise zur Erzeugung von zweidimensionalen Projektionsaufnahmen oder auch zur Erzeugung von dreidimensionalen Niedrigkontrast-Aufnahmen eingesetzt. Bei letzterem wird der Patient mit einem aufgefächerten Röntgenstrahl bestrahlt und die vom Strahlendetektor empfangenen Signale werden zur Erzeugung eines dreidimensionalen Bildes ausgewertet. Dieses bildgebende Verfahren ist auch unter dem Namen 3D-Cone Beam Rekonstruktion bekannt.

Als Tragarm werden vorzugsweise C- oder U-förmige mechanische Tragkonstruktionen verwendet, die zu einer Seite hin offen sind, so dass der Tragarm die zwischen dem Strahlendetektor und der Strahlenquelle angeordnete Patientenliege bogenartig umspannt.

Zweckdienlicherweise ist der Tragarm derart gelagert und ansteuerbar, dass sowohl für die Angularbewegung als auch für die Orbitalbewegung ein Drehwinkel von mindestens 180° ausführbar ist. Damit lassen sich umfassende Bildinformationen insbesondere auch für eine dreidimensionale Bilderzeugung erhalten. Bei der Verwendung eines Röntgen-Fächerstrahls für das so genannte 3D-Cone Beam-Verfahren beträgt die Drehbewegung für die Angular- und Orbitalbewegung mindestens 180° zuzüglich der Fächeraufweitung des Röntgenstrahls.

Gemäß einer bevorzugten Ausgestaltung ist weiterhin vorgesehen, dass der Tragarm am Partikelstrahler drehbar gelagert ist. Der Partikelstrahler und das Diagnosegerät bilden daher eine miteinander verbundene einheitliche Baueinheit aus. Durch die bauliche Verbindung ist die Relativposition zwischen der Strahlenquelle und dem Strahlendetektor bezüglich des Isozentrums des Partikelstrahlers stets exakt definiert.

Gemäß einer bevorzugten alternativen Ausführungsform ist der Tragarm von einem mehrachsigen, insbesondere sechsachsigen Roboterarm gehalten. Durch die Führung über einen mehrachsigen Roboterarm ist eine freie Bewegung im Raum ohne oder nahezu ohne Beschränkungen ermöglicht, so dass individuelle Anforderungen problemlos berücksichtigt werden können. In beiden Varianten ist eine Tragstruktur des Diagnosegeräts ortsfest im Raum angeordnet, lediglich der Tragarm ist im Raum frei verfahrbar.

Um nach der Verifikation der Position des Tumors den Patienten exakt in die Sollposition zu verfahren, ist gemäß einer zweckdienlichen Ausgestaltung vorgesehen, dass die Patientenliege gesteuert in eine vordefinierte Bestrahlungsposition verfahrbar ist.

Zu diesem Zweck ist insbesondere eine gemeinsame Steuereinheit vorgesehen, die zwei Geräte, nämlich das Diagnosegerät und die Patientenlagerungsvorrichtung, aufeinander abgestimmt steuert. Und zwar derart, dass unter Berücksichtigung des Isozentrums des Partikelstrahls und unter Berücksichtigung der mit Hilfe des Diagnosegeräts ermittelten Tumorposition die Patientenliege mit dem darauf immobilisierten Patienten in die erforderliche Bestrahlungsposition verfahren wird. Die Bestimmung der Position des Tumors anhand der Röntgenbilder erfolgt hier entweder automatisch durch geeignete automatische Bilderkennungsverfahren oder manuell durch geschultes medizinisches Personal, das der Steuereinheit über ein geeignetes Eingabegerät die Position des Tumors mitteilt.

Zum Teil sind mehrere Austrittsfenster für den Partikelstrahl unter vorgegebenen Winkeln relativ zur Patientenposition vorgesehen. Aufgrund der Verfahrbarkeit des Diagnosegeräts im Raum um die in der Behandlungsposition befindliche Patientenliege ist das gleiche Diagnosegerät auch für Strahlentherapieanwendungen mit mehreren Austrittsfenstern für den Partikelstrahl geeignet. Vorzugsweise wird daher das Diagnosegerät eingesetzt in Kombination mit mehreren unter bestimmten Winkeln angeordneten Austrittsfenstern für Partikelstrahlen mit jeweils im Raum feststehenden Strahlaustritten.

Ausführungsbeispiele der Erfindung werden im Folgenden anhand der Figuren näher erläutert. Es zeigen jeweils in schematischen Darstellungen:
- FIG 1 bis 3: eine Strahlentherapieanordnung mit einem drehbar gelagerten Röntgen-Diagnosegerät mit einem C-bogenartigen Tragarm in unterschiedlichen Positionen des Tragarms für unterschiedliche Bestrahlungspositionen und
- FIG 4,5: eine alternative Ausführungsform der Strahlentherapieanordnung mit einem an einem mehrachsigen Roboterarm befestigten Tragarm.

In den Figuren sind gleich wirkende Teile mit den gleichen Bezugszeichen versehen.

Die medizinische Strahlentherapieanordnung umfasst in allen Ausführungsvarianten einen Teilchenbeschleuniger zur Erzeugung eines Partikelstrahls bestehend aus Schwerionen, im Folgenden als Partikelstrahler bezeichnet. Dieser umfasst eine Röhre 2 mit einem vorderseitigen Austrittsfenster 4, aus dem an einer vordefinierten Position ein Partikelstrahl 6 während des Betriebs austritt. Die Röhre 2 mit dem Austrittsfenster 4 ist im Raum ortsfest angeordnet. Alternativ zu den dargestellten Ausführungsbeispielen sind im Raum unter definierten Winkeln mehrere feststehende Austrittsfenster 4 angeordnet.

Die Anordnung weist weiterhin ein Röntgen-Diagnosegerät 8 mit einem Tragarm 10A,10B auf, an dem an gegenüberliegenden Positionen jeweils eine Röntgenstrahlenquelle 12 und ein Röntgenstrahlendetektor 14 angeordnet sind.

Weiterhin umfasst die Strahlentherapieanordnung eine Patientenlagerungsvorrichtung 16 mit einer Patientenliege 18, welche über einen ersten mehrachsigen Roboterarm 20 gesteuert verfahrbar ist. Unter Patientenliege 18A,18B wird hier allgemein eine Vorrichtung verstanden, auf der ein Patient 22 in eine für die Strahlentherapie vorgesehene Position gebracht wird. Dies kann sowohl eine sitzende als auch eine liegende Position sein. Unter Patientenliege 18A,18B wird daher sowohl eine Liege für eine liegende Position des Patienten 22 als auch ein stuhlartiges Gebilde für eine sitzende Position des Patienten 22 verstanden.

Von besonderer Bedeutung bei der Strahlentherapieanordnung ist die besondere Ausgestaltung und Anordnung des Röntgen-Diagnosegeräts 8 in Kombination mit dem Austrittsfenster 4 derart, dass eine Röntgenaufnahme zur Bestimmung oder Verifikation der Position eines Tumors in der für die Strahlentherapie vorgesehenen Behandlungsposition des Patienten 22 durchführbar ist. Das Röntgen-Diagnosegerät 8 ist hierbei prinzipiell sowohl für 2D-Projektionsaufnahmen als auch für 3D-Niedrigkontrastaufnahmen und zur Erzeugung von so genannten 3D-Cone-Beam-Bildern geeignet.

In den Ausführungsbeispielen ist jeweils ein Röntgen-Fächerstrahl 24 dargestellt, welcher zur Erzeugung der 3D-Cone-Beam-Bilder, also zur Erzeugung von dreidimensionalen Bildern des zu bestrahlenden Tumors vorgesehen ist. Die Bilderzeugung und Bildauswertung erfolgt in an sich bekannter Weise. Für die Erzeugung von 3D-Röntgenbildern ist es erforderlich, dass der Fächerstrahl 24 um mindestens 180° zuzüglich des Fächerwinkels um den Patienten 22 verfahrbar ist. Von besonderer Bedeutung bei der vorliegenden Strahlentherapieanordnung ist, dass unabhängig von der jeweiligen Bestrahlungsposition des Patienten 22 eine Positionsverifikation des Tumors mit ein und demselben Diagnosegerät durchführbar ist. Die Positionsverifikation ist daher unabhängig davon, ob der Patient 22 in liegender oder in sitzender Position, ob in Längsrichtung zum Partikelstrahl 6 oder quer hierzu ausgerichtet ist. Maßgebend für diese Variabilität ist die im Wesentlichen frei im Raum verfahrbare Anordnung des Röntgen-Diagnosegeräts 8.

Der Tragarm 10A,10B ist zu einer Seite hin offen, also etwa U-förmig oder C-förmig ausgebildet, so dass er problemlos über den Patient verfahrbar ist, so dass dieser zwischen die Strahlenquelle 12 und den Strahlendetektor 14 positioniert ist. Für die Drehbarkeit um mehr als 180° um den Patienten 22 herum kann der Tragarm 10A,10B zum einen eine Angularbewegung um die durch den Partikelstrahl 6 definierte Längsachse ausführen. Die Angularbewegung ist durch einen Doppelpfeil 26 angedeutet. Zum anderen ist der Tragarm 10A,10B in der Lage, eine Orbitalbewegung um eine Achse senkrecht zum Partikelstrahl 6 auszuführen. Diese Orbitalbewegung ist durch einen weiteren Doppelpfeil 28 angedeutet.

Hat der Patient 22 eine definierte Behandlungsposition eingenommen, so vollführt das Diagnosegerät 8 in der Regel eine 180°-Drehung (zzgl. des Fächerwinkels des Röntgenstrahls) in nur einer der beiden Bewegungsrichtungen, also entweder eine Angularbewegung 26 um die Längsachse oder eine Orbitalbewegung 28 um eine senkrecht zur Längsachse stehende weitere Drehachse. Die Lage dieser weiteren Drehachse im Raum ist hierbei variabel und hängt von der jeweiligen angularen Drehposition des Tragarms 10A,10B ab.

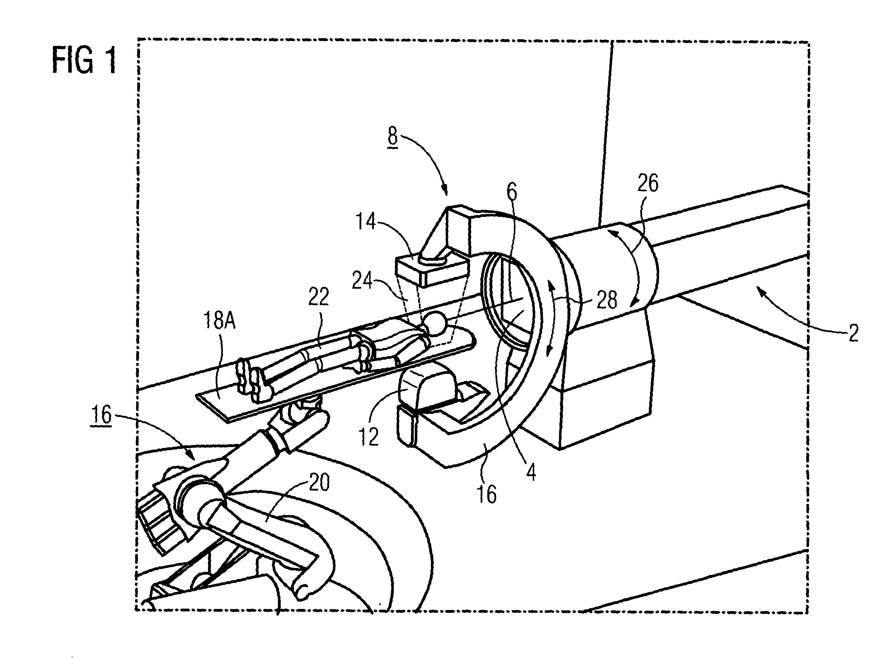

Beim Ausführungsbeispiel gemäß den FIG 1 bis 3 ist der Tragarm 10A als ein C-Bogen ausgebildet, welcher an seinen Bogenenden jeweils die Strahlenquelle 12 und den Detektor 14 aufweist. Der Tragarm 10A ist zur Ausführung der Angularbewegung 26 über einen Drehring 30 gelagert, welcher ringförmig an der Röhre 2 umläuft. Über diesen Drehring 30 ist daher der Tragarm 10A um die durch den Partikelstrahl 6 gebildete Längsachse drehbar. Zur Ausführung der Orbitalbewegung 28 ist der Tragarm 10A am Drehring 30 selbst wiederum verschieblich gelagert, beispielsweise geführt durch eine Führungsschiene oder eine ineinandergreifende Verzahnung zwischen dem Drehring 30 und dem Tragarm 10A. Durch diese Maßnahme lässt sich in jeder Angular-Drehstellung eine Orbital-Drehung um 180° ausführen. Umgekehrt lässt sich in jeder Orbital-Drehstellung auch eine Angular-Drehbewegung 26 um mindestens 180° ausführen. Bei der gewählten Konstruktion sind prinzipiell jeweils Drehbewegungen um 360° möglich. Die Drehbewegungen werden im Wesentlichen durch die Position der Patientenliege 18A,18B begrenzt.
Im Ausführungsbeispiel der FIG 1 befindet sich der Patient 22 in einer längs zum Partikelstrahl 6 gerichteten liegenden Behandlungsposition zur Behandlung eines Gehirntumors. Der Tragarm 10A wird zur 3D-Bildverifikation der Position des Gehirntumors durch Verdrehen des Drehrings 30 in Angularrichtung 26 um mindestens 180° um den Kopf des Patienten 22 gedreht.

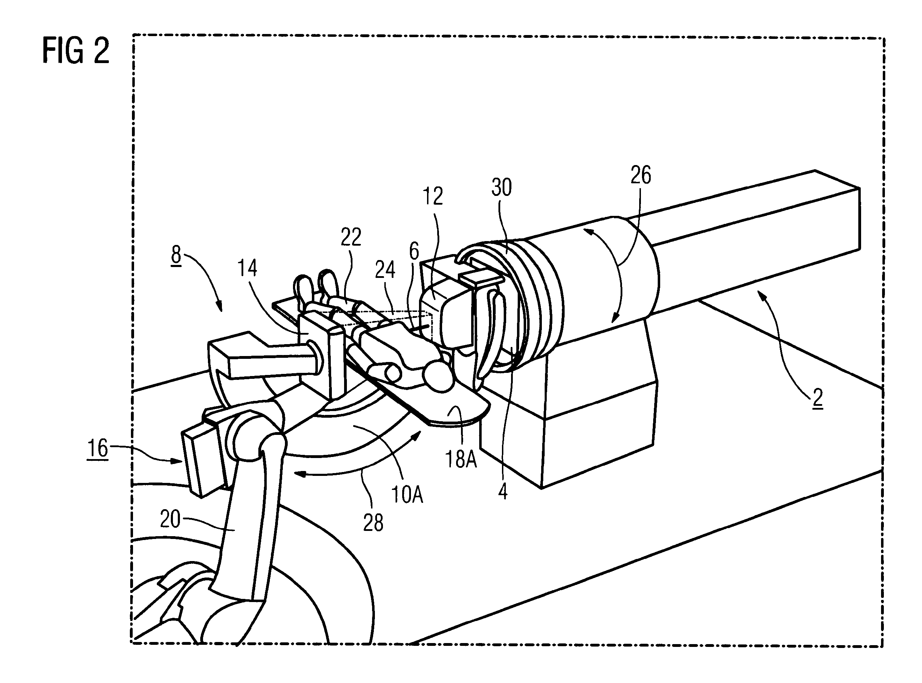

Bei der in FIG 2 dargestellten Bestrahlungsposition befindet sich der Patient 22 quer zum Partikelstrahl 6 in einer liegenden Position. Der Tragarm 10A ist hierbei in einer festen Angularposition orientiert. Zur Röntgenaufnahme wird der Tragarm 10A um mindestens 180° in Orbitalrichtung 28 um den Rumpf des Patienten 22 geschwenkt. Eine Drehbewegung in Angularrichtung 26 erfolgt hierbei nicht.

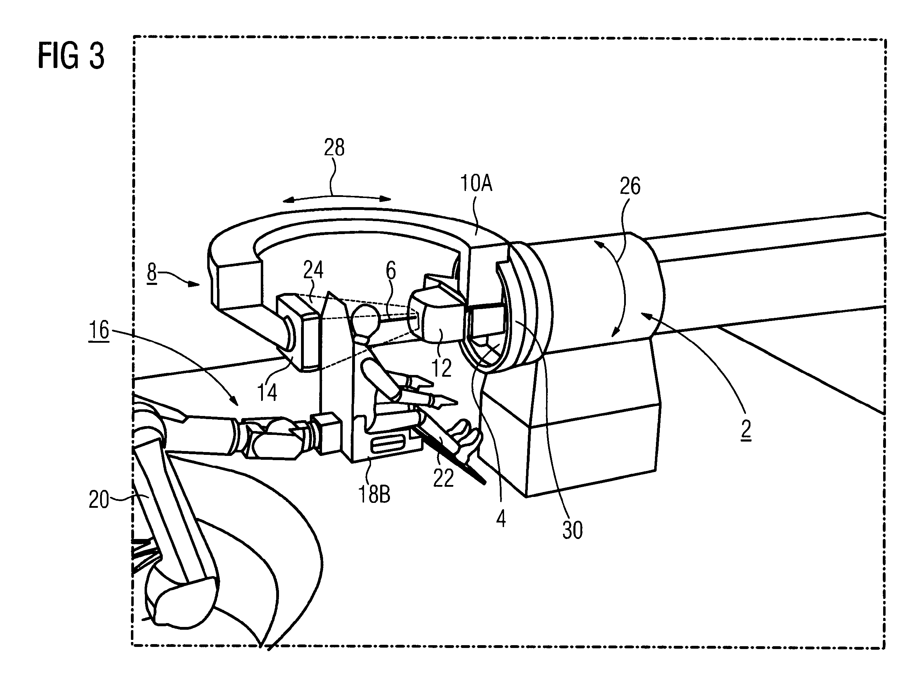

Bei der Behandlungssituation gemäß FIG 3 befindet sich der Patient 22 in einer sitzenden Behandlungsposition. Zur Aufnahme des Röntgenbilds erfolgt hierbei wiederum lediglich eine Bewegung des Tragarms 10A in Orbitalrichtung 28.

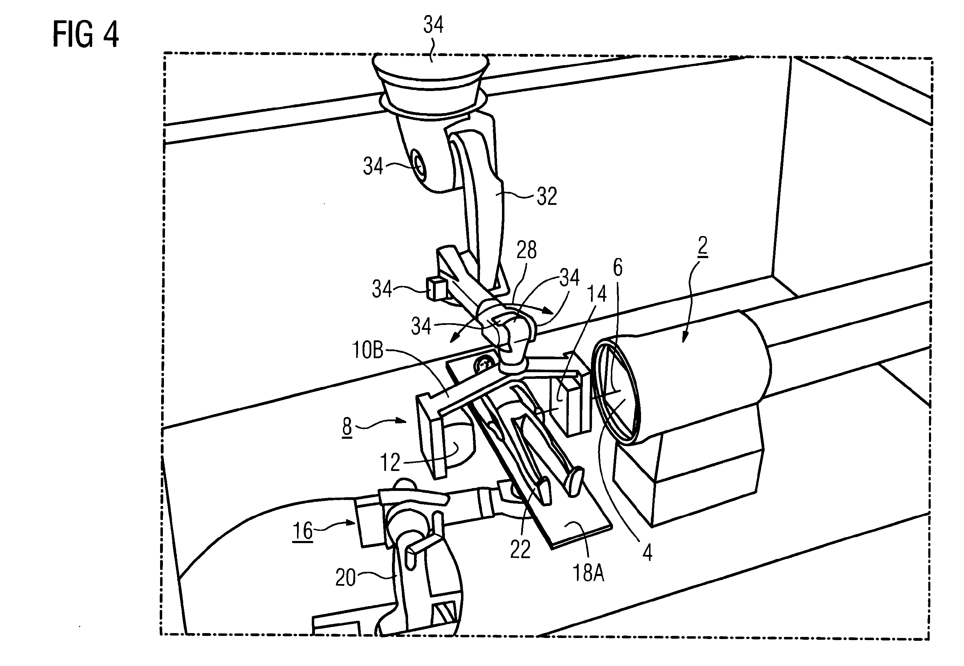

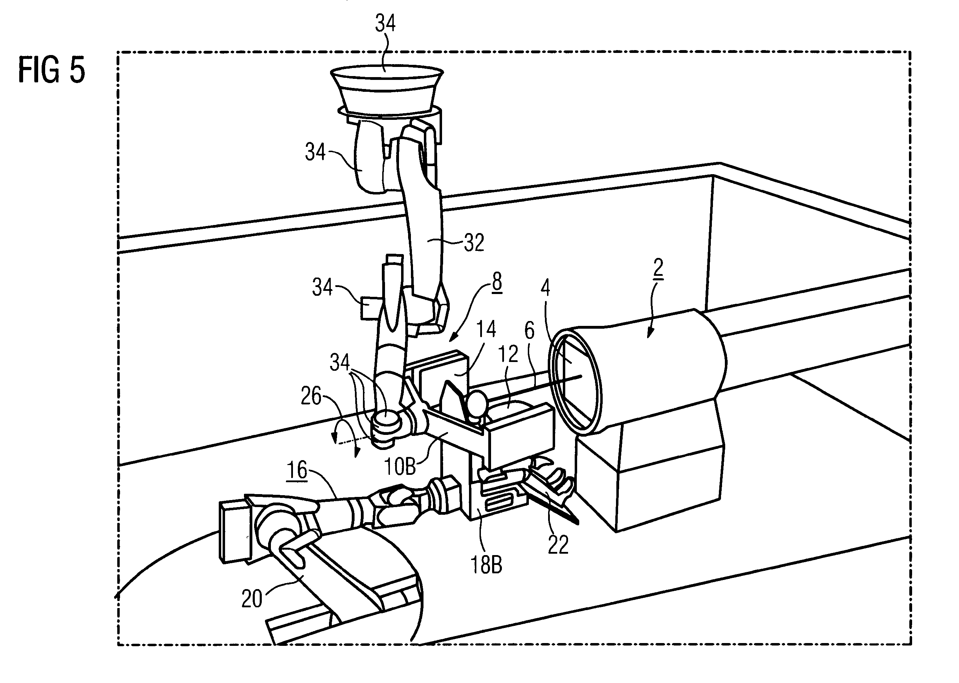

Im Unterschied zu der ersten in den in FIG 1 bis 3 dargestellten Ausführungsvariante ist bei der zweiten Ausführungsvariante gemäß den FIG 4 und 5 der Tragarm 10B an einem insbesondere 6-achsigen zweiten Roboterarm 32 befestigt. Der zweite Roboterarm 32 ist im Ausführungsbeispiel an einer Raumdecke befestigt. Durch die mehrachsige Ausführung des zweiten Roboterarms 32 lässt sich der Tragarm 10B an beliebigen, vom zweiten Roboterarm 32 zugänglichen Stellen im Raum positionieren. Zur Durchführung der angularen Drehbewegung 26 bzw. der orbitalen Drehbewegung 28 weist der zweite Roboterarm 32 mehrere Drehgelenke 34 auf. Auch hier wird zur Aufnahme des Röntgenbilds in Abhängigkeit der jeweiligen Behandlungsposition, in der sich der Patient 22 befindet, jeweils eine mindestens 180°-Drehung entweder in Orbitalrichtung 28 oder in Angularrichtung 26 vorgenommen.

Das Röntgen-Diagnosegerät 8, die Patientenlagerungsvorrichtung 16 sowie gegebenenfalls der Teilchenbeschleuniger werden vorzugsweise von einer gemeinsamen Steuereinheit aus aufeinander abgestimmt betrieben. Zur Durchführung der Strahlentherapie ist vorzugsweise vorgesehen, dass der Patient 22 in eine vorläufige Behandlungsposition für die Strahlentherapie gebracht wird. Hierzu wird er zunächst auf der Patientenliege 18A,18B immobilisiert und die Patientenliege 18A,18B wird mit Hilfe des ersten Roboterarms 20 in die gewünschte vorläufige Behandlungsposition verfahren. In dieser Position wird die Position des Tumors mit Hilfe des Röntgengeräts 8 bestimmt und verifiziert. Der immobilisierte Patient 22 wird über die Patientenlagerungsvorrichtung 8 insbesondere automatisch und gesteuert in die optimale Bestrahlungsposition verfahren, so dass der Tumor im Isozentrum positioniert ist. Die richtige Positionierung wird mit dem Röntgengerät 8 verifiziert.

Die Bestimmung der Position des Tumors erfolgt hierbei entweder automatisch oder durch Auswertung der ermittelten Bilder durch medizinisches Fachpersonal. Nach der Positionierung des Patienten 22 wird der Partikelstrahl 6 wird erzeugt und der Patient 22 bestrahlt. In den Figuren ist der Partikelstrahl 6 jeweils gestrichelt dargestellt, um anzudeuten, dass die Bestrahlung mit dem Partikelstrahl 6 in der Regel nach der Durchführung der Röntgenaufnahmen erfolgt.

Mit Hilfe der hier nicht näher dargestellten Steuer-, Kontroll- und Überwachungseinheit wird der Patient 22 vorzugsweise automatisch in die richtige Behandlungsposition für die Strahlenbehandlung verfahren.

## Patentansprüche

1. Medizinische Ionenstrahlentherapieanordnung mit einem feststehenden Austrittsfenster (4) für einen Partikelstrahl (6), mit einer Patientenlagerungsvorrichtung (16), umfassend eine Patientenliege (18A,18B), die vor das Austrittsfenster (4) in eine zur Bestrahlung eines Patienten (22) geeignete Bestrahlungsposition mithilfe eines mehrachsigen Roboterarms (20) gesteuert verfahrbar ist, und mit einem Diagnosegerät (8) zur Bestimmung der Position eines zu bestrahlenden Tumors,
**dadurch gekennzeichnet, dass**
das Diagnosegerät (8) eine Strahlenquelle (12) und einen gegenüberliegenden Strahlendetektor (14) aufweist, die an einem gemeinsamen Tragarm (10A,10B) befestigt sind, wobei der Tragarm (10A,10B) derart gelagert und ansteuerbar ist, dass sowohl eine Angularbewegung (26) um die durch den Partikelstrahl (6) definierte Längsachse als auch eine Orbitalbewegung (28) um eine zur Längsachse senkrechte Achse ausführbar ist, so dass die Strahlenquelle (12) und der Strahlendetektor (14) im Raum um die in der jeweiligen Bestrahlungsposition befindliche Patientenliege (18A,18B) verfahrbar sind.

2. Ionenstrahlentherapieeinrichtung nach Anspruch 1, bei der das Diagnosegerät (8) derart ausgebildet ist, dass die Tumorposition anhand von 2-dimensionalen Projektionsaufnahmen oder anhand von 3-dimensionalen Schnittbildaufnahmen in jeder beliebigen Bestrahlungsposition des Patienten bestimmbar ist.

3. Ionenstrahlentherapieanordnung nach Anspruch 1 oder 2, bei der das Diagnosegerät (8) derart ausgebildet ist, dass sowohl bei der Verwendung einer Patientenliege in Form einer Liege (18A) für eine liegende Bestrahlungsposition als auch in Form eines Stuhls (18B) für eine sitzende Bestrahlungsposition die Strahlenquelle (12) und der Strahlendetektor (14) frei im Raum um die Patientenliege (18A,18B) verfahrbar sind.

4. Ionenstrahlentherapieanordnung nach einem der vorhergehenden Ansprüche 4, bei der der Tragarm (10A,10B) C-förmig oder U-förmig ausgebildet ist.

5. Ionenstrahlentherapieanordnung nach einem der vorgehenden Ansprüche, bei der eine Drehbewegung des Tragarms (10A,10B) sowohl für die Angularbewegung (26) als auch für die Orbitalbewegung (28) um mindestens 180° ausführbar ist.

6. Ionenstrahlentherapieanordnung nach einem der vorhergehenden Ansprüche, bei der der Tragarm (10A) an einer Vorrichtung zur Erzeugung des Partikelstrahls drehbar gelagert ist.

7. Ionenstrahltherapieanordnung nach einem der vorhergehenden Ansprüche, mit einer durch das Austrittsfenster (4) verschlossenen Röhre (2) zur Führung des Partikelstrahls (6), wobei der Tragarm (10A) zur Ausführung der Angularbewegung (26) an der Röhre (2) um diese drehbar gelagert ist.

8. Ionenstrahlentherapieanordnung nach einem der Ansprüche 1 bis 5, bei der der Tragarm (10B) von einem mehrachsigen Roboterarm gehalten ist.

9. Ionenstrahlentherapieanordnung nach einem der vorhergehenden Ansprüche, bei der die Patientenliege (22) gesteuert in eine vordefinierte Bestrahlungsposition verfahrbar ist.

10. Ionenstrahlentherapieanordnung nach einem der vorhergehenden Ansprüche, bei der das Austrittsfenster (4) ortsfest in einem Raum angeordnet und Teil einer Vorrichtung zur Erzeugung eines Ionen-Partikelstrahls (6) ist.

11. Ionenstrahlentherapieanordnung nach einem der vorhergehenden Ansprüche, bei der mehrere feststehende Austrittsfenster (4) vorgesehen sind.

## Claims

1. Medical ion radiation therapy arrangement having a fixed discharge window (4) for a particle beam (6), having a patient support device (16), comprising a patient couch (18A, 18B) which can be moved in a controlled manner by means of a multiple-axle robot arm (20) in front of the discharge window (4) into a radiation position suitable for radiating a patient (22), and having a diagnostic device (8) for determining the position of a tumour to be radiated,
**characterised in that**
the diagnostic device (8) has a radiation source (12) and an opposed radiation detector (14) which are attached to a common support arm (10A, 10B), the support arm (10A, 10B) being mounted and controllable such that both an angular movement (26) about the longitudinal axis defined by the particle beam (6) and an orbital movement (28) about an axis perpendicular to the longitudinal axis can be performed, so that the radiation source (12) and the radiation detector (14) can be moved in the room around the patient couch (18A, 18B) located in the respective radiation position.

2. Ion radiation therapy device according to claim 1,
wherein the diagnostic device (8) is designed such that the position of the tumour can be determined on the basis of 2-dimensional projection recordings or on the basis of 3-dimensional sectional view recordings in any radiation position of the patient.

3. Ion radiation therapy arrangement according to claim 1 or 2,
wherein the diagnostic device (8) is designed such that both when using a patient couch in the form of a couch (18A) for a recumbent radiation position as well as in the form of a chair (18B) for a sitting radiation position the radiation source (12) and the radiation detector (14) can be moved freely in the room around the patient couch (18A, 18B).

4. Ion radiation therapy arrangement according to one of the preceding claims 4, wherein the support arm (10A, 10B) is designed to be C-shaped or U-shaped.

5. Ion radiation therapy arrangement according to one of the preceding claims, wherein a rotational movement of the support arm (10A, 10B) can be performed both for the angular movement (26) and for the orbital movement (28) about at least 180°.

6. Ion radiation therapy arrangement according to one of the preceding claims, wherein the support arm (10A) is rotatably mounted on an apparatus for generating the particle beam.

7. Ion radiation therapy arrangement according to one of the preceding claims, having a tube (2) closed by the discharge window (4) for guiding the particle beam (6), wherein the support arm (10A) is rotatably mounted about the tube (2) to perform the angular movement (26) on said tube (2).

8. Ion radiation therapy arrangement according to one of claims 1 to 5, wherein the support arm (10B) is held by a multiple-axle robot arm.

9. Ion radiation therapy arrangement according to one of the preceding claims, wherein the patient couch (22) can be moved in a controlled manner into a predefined radiation position.

10. Ion radiation therapy arrangement according to one of the preceding claims, wherein the discharge window (4) is arranged in a fixed position in a room and is part of an apparatus for generating an ion particle beam (6).

11. Ion radiation therapy arrangement according to one of the preceding claims, wherein a plurality of fixed discharge windows (4) is provided.

## Revendications

1. Ensemble médical de radiothérapie par rayons ioniques, avec une fenêtre de sortie fixe (4) pour un faisceau de particules (6), avec un dispositif de positionnement du patient (16), comprenant une table de patient (18A, 18B) qui, commandée à l'aide d'un bras de robot multiaxial (20), peut être déplacée devant la fenêtre de sortie (4) dans une position d'irradiation appropriée à l'irradiation d'un patient (22), et avec un appareil de diagnostic (8) pour déterminer la position d'une tumeur à irradier,
**caractérisé en ce que**
l'appareil de diagnostic (8) comporte une source de rayonnement (12) et un détecteur de rayonnement (14) placé en regard, qui sont fixés sur un bras porteur (10A, 10B) commun, ledit bras porteur (10A, 10B) étant monté, et pouvant être commandé de manière à pouvoir effectuer aussi bien un mouvement angulaire (26) autour de l'axe longitudinal défini par le faisceau de particules (6) qu'un mouvement orbital (28) autour d'un axe perpendiculaire à l'axe longitudinal, permettant ainsi de déplacer la source de rayonnement (12) et le détecteur de rayonnement (14) dans l'espace autour de la table de patient (18A, 18B) dans sa position d'irradiation respective.

2. Dispositif de radiothérapie par rayons ioniques selon la revendication 1, dans lequel l'appareil de diagnostic (8) est conçu de telle manière que la position de la tumeur pourra être déterminée à l'aide d'images de projection à 2 dimensions ou à l'aide d'images en coupe à 3 dimensions dans n'importe quelle position d'irradiation du patient.

3. Dispositif de radiothérapie par rayons ioniques selon la revendication 1 ou 2, dans lequel l'appareil de diagnostic (8) est conçu de telle manière qu'aussi bien dans le cas de l'utilisation d'une table de patient sous forme d'une couchette (18A) pour une position d'irradiation allongée que sous forme d'un fauteuil (18B) pour une position d'irradiation assise, la source de rayonnement (12) et le détecteur de rayonnement (14) pourront être déplacés librement dans l'espace autour de la table de patient (18A, 18B).

4. Dispositif de radiothérapie par rayons ioniques selon l'une des revendications précédentes, dans lequel le bras porteur (10A, 10B) a une conformation en C ou en U.

5. Dispositif de radiothérapie par rayons ioniques selon l'une des revendications précédentes, dans lequel un mouvement de rotation du bras porteur (10A, 10B), aussi bien pour le mouvement angulaire (26) que pour le mouvement orbital (28), d'au moins 180° peut être effectué.

6. Dispositif de radiothérapie par rayons ioniques selon l'une des revendications précédentes, dans lequel le bras porteur (10A) est monté tournant sur un dispositif de production du faisceau de particules.

7. Dispositif de radiothérapie par rayons ioniques selon l'une des revendications précédentes, avec un tube (2) fermé par la fenêtre de sortie (4) et destiné à guider le faisceau de particules (6), le bras porteur (10A), pour effectuer le mouvement angulaire (26), étant monté sur ledit tube (2) de manière à pouvoir tourner autour de celui-ci.

8. Dispositif de radiothérapie par rayons ioniques selon l'une des revendications 1 à 5, dans lequel le bras porteur (10B) est tenu par un bras de robot multiaxial.

9. Dispositif de radiothérapie par rayons ioniques selon l'une des revendications précédentes, dans lequel la table de patient (22) peut être déplacée, de manière commandée, dans une position d'irradiation prédéfinie.

10. Dispositif de radiothérapie par rayons ioniques selon l'une des revendications précédentes, dans lequel la fenêtre de sortie (4) est disposée en position fixe dans un espace et fait partie d'un dispositif de production d'un faisceau de particules ioniques (6).

11. Dispositif de radiothérapie par rayons ioniques selon l'une des revendications précédentes, dans lequel plusieurs fenêtres de sortie fixes (4) sont prévues.
